# EUROPEAN PATENT SPECIFICATION

(11) **EP 1 589 875 B1**
(45) Date of publication and mention of the grant of the patent: **14.03.2012**
(21) Application number: 04700416.3
(22) Date of filing: 07.01.2004
(51) Int. Cl.: A61B 5/16

(54) **ASSESSMENT OF COGNITIVE IMPAIRMENT**
BEURTEILUNG KOGNITIVER BEEINTRÄCHTIGUNG
EVALUATION DE LA DEFICIENCE INTELLECTUELLE

(30) Priority: 07.01.2003 AU 2003900035
(43) Date of publication of application: 02.11.2005
(73) Proprietor: MONASH UNIVERSITY, Clayton, VIC 3168 (AU)
(72) Inventor: YELLAND, Gregory, Glen Waverley, Victoria 3150 (AU); ROBINSON, Stephen, Glen Waverley, Victoria 3150 (AU); FRIEDMAN, Timothy, McKinnon, Victoria 3204 (AU); HUTCHISON, Christopher, Hampton, Victoria 3188 (AU)
(74) Representative: Mabey, Katherine Frances
(86) International application number: PCT/AU2004/000008
(87) International publication number: WO 2004/060164

(56) References cited:
- EP-A2- 0 114 037
- EP-B1- 0 578 236
- WO-A-00/44283
- WO-A1-03/075762
- DD-A1- 272 408
- RU-C1- 2 138 199
- US-A- 5 325 136
- US-A- 5 911 581
- US-A- 5 911 581
- US-A- 6 053 739
- US-A- 6 053 739
- US-A1- 2002 192 624
- US-A1- 2003 109 799
- DATABASE WPI Week 200038, Derwent Publications Ltd., London, GB; Class P31, AN 2000-440057, XP008100184 & RU 2 138 199 C1 (MUKHINA) 27 September 1999

## Description

### Field of Invention

The invention relates to a system of assessing cognitive impairment. More particularly, it relates to a system of assessing cognitive impairment that involves the presentation of a visual stimulus to a user and subsequent assessment of the user's response.

### Background to the Invention

There are a number of neuropsychological and computer based tests that have been developed to diagnose and measure cognitive impairment. Typically, methods of assessing subjects for cognitive impairment have allowed subjects to respond at their own leisure. This was done primarily because the principal aim is to estimate cognitive processing speed independently of motor ability. Monitoring the time taken for the user to respond was therefore seen as introducing an error factor into any estimation of cognitive impairment.

A major disadvantage of this type of test for detecting cognitive impairment was that it tended to suffer from a lack of sensitivity making it unsuitable for the detection of subtle cognitive impairment and small variations in cognitive ability over time.

Existing methods of detecting cognitive impairment tend to be time-consuming methods which involve gradually changing the duration over which a stimulus is presented to a subject until a predetermined error rate is achieved. These methods have a relatively limited statistical power because they only produce a single score for each test subject. Further, the existing methods generally use masks that are ineffective at completely blocking out an afterimage of a previously presented stimulus. Moreover, existing methods do not have the capacity to accommodate the possibility that impaired vision may influence the performance of test subjects. Existing methods also tend to result in a learning effect which is to the detriment of the test being performed because they can not be repeated on the subject without a considerable interval between tests.

These methods also tend to require specially trained personnel to assess the results and to evaluate the subject's performance, resulting in a subjective evaluation. This is disadvantageous because the introduction of a subjective assessment creates a potential for error which is further magnified when different psychologists become involved. Where tests are conducted on a subject having characteristics which differ from the norm, assessment becomes very complicated because premorbid intelligence, depression or gender bias may influence the results.

WO 00/44283 discloses remote computer-implemented methods for cognitive and perceptual testing. US 5911581 discloses an interactive computer program for measuring and analysing mental activity. RU C12138199 discloses a method of assessment of efficiency of man psychical functions and emotional stability. US 6053739 discloses a system for measurement of attention span and attention deficicits.

The discussion of the background to the invention herein is included to explain the context of the invention. It is not to be taken as an admission or a suggestion that any of the material referred to was published, known or was part of the common general knowledge as at the priority date of the claims appended hereto.

It is an object of the present invention to overcome, alleviate or minimise one or more of the problems present in prior art methods of measuring cognitive impairment.

### Summary of the Invention

The present invention provides a system for assessing cognitive impairment of a user in accordance with claim 1.

Also disclosed is a method for assessing cognitive impairment of a user, including the steps of:
(a) presenting a visual test stimulus to the user for a pre-determined test stimulus exposure duration;
(b) masking the test stimulus;
(c) measuring a response from the user, the response providing Information about the user's perception of a characteristic of the test stimulus together with the time taken for the user to respond;
(d) repeating steps (a) to (c) to develop a user profile; and
(e) assessing cognitive impairment in the user by comparing the user profile with a reference profile.

Preferably, masking the test stimulus occurs by replacing it with a mask. Alternatively, masking may occur by blocking the user's line of vision to the test stimulus using a mask. Preferably, step (a) includes the step of presenting a focal point stimulus to the user before presenting the visual test stimulus to the user.

The user profile may include a response curve which is indicative of the user's response as a function of test stimulus exposure duration. The response curve may therefore provide response time as function of test stimulus exposure duration, error rate as a function of test stimulus exposure duration, or any other measure of response as a function of test stimulus exposure duration.

Step (d) may include repeating steps (a) to (c) for a range of pre-determined test stimulus exposure durations. Preferably, for each of the users assessed the predetermined test stimulus exposure durations are the same. This will allow for a relative comparison between each of the users assessed.

In one form, the method further includes the step of calculating a representative error rate which represents the proportion of inaccurate responses provided for each of the pre-determined test stimulus exposure durations. This representative error rate can then be included in the user profile and used to assess the subject's degree of cognitive impairment.

The user profile preferably includes an error rate curve which charts the user's average error rate relative to the pre-determined test stimulus exposure durations. This facilitates comparison of the user's profile with the profile of a reference group such as a group of cognitively normal individuals or another profile such as one which has been generated during a previous assessment of the user. A deviation of the user's error rate curve from the reference group's error rate curve can be used to assess a degree of cognitive impairment of the user, relative to that of the reference group or the previous profiles of the user.

The representative error rate may, for example, be calculated from a mean of the error of each response at the pre-determined test stimulus exposure duration.

Additionally or alternatively, the method includes the step of calculating an average response time for each of the pre-determined test stimulus exposure durations. Where the average response time is calculated, the user profile may optionally include a response time curve charting the mean response rate relative to the pre-determined test stimulus exposure duration.

In a preferred form of the method, the pre-determined test stimulus exposure durations are equally spaced. It is also preferred that the test stimulus exposure durations are the same for all subjects who are tested.

The reference profile may be generated in any suitable manner. Preferably, the reference profile is generated using data obtained by testing a population of cognitively normal individuals. It may also be generated from the results of a previous assessment of the user.

The test stimulus may be presented to the user for a test stimulus exposure duration of between 10ms and 300ms. The user makes a response which relates to a characteristic of each test stimulus. The characteristic may be any suitable feature such as a line, colour or textured aspect of the visual test stimulus. Preferably the response is selected from one of two choices; one of which is correct and the other incorrect. However, it is to be understood that a larger number of choices may be provided. Each of the test stimuli are presented separately to the user in step (a). Preferably, each of the stimuli is presented an equal number of times.

A mask is used with the present invention. A particularly advantageous mask to use is an image including at least one full circle, especially when the visual stimulus includes one or more lines since curves and lines have different effects on the visual system. Preferably the image includes a plurality of full circles or parts thereof when the test stimulus consists of linear (non-curved) features.

Also disclosed is a method for assessing visual impairment of a user, including the steps of:
(i) presenting a visual test stimulus to the user for a pre-determined test stimulus exposure duration;
(ii) masking the test stimulus;
(iii) measuring a response from the user, the response providing information about the user's perception of the test stimulus;
(iv) repeating steps (i) to (iii) using the same pre-determined test stimulus exposure duration to develop a user profile; and
(v) assessing visual impairment of the user by comparing the user profile with a reference profile.

Preferably, step (i) includes the step of presenting a focal point stimulus to the user before presenting the visual test stimulus to the user. Preferably steps (i) to (iii) are repeated using the same pre-determined test stimulus exposure duration. In a preferred method the measured response includes the time taken for the user to respond to the visual test stimulus.

Also disclosed is a method for assessing cognitive impairment of a user, including the steps of:
(i) presenting a visual test stimulus to the user for a pre-determined test stimulus exposure duration;
(ii) masking the test stimulus;
(iii) measuring a response from the user, the response including:
   (a) a response time; and
   (b) a correct or incorrect indication of the visual test stimulus;
(iv) repeating steps (i) to (iii) to develop a user profile; and
(v) assessing cognitive impairment of the user by comparing the user profile to a reference profile.

Preferably, the system of the present invention also includes focal point presentation means for presenting a focal point stimulus to the user.

In embodiments the mask is a mask for masking a visual test stimulus, the mask including an image having a plurality of filled circles or parts thereof.

### Brief Description of Drawings

The invention will now be described in further detail by reference to the enclosed drawings illustrating example forms of the invention. It is to be understood that the particularity of the drawings does not supersede the generality of the preceding description of the invention. In the drawings:
Figures 1A, 1B and 1C are various screen illustrations presented to a user in one embodiment of the present invention. Figure 1A illustrates a focal point stimulus which is presented to the user before the user is tested. Figure 1 B shows examples of visual test stimuli. Figure 1C shows an example of a mask.
Figures 2A-C, 3A-C and 4A-C illustrate different forms of visual test stimuli and masks presented to the user in various embodiments of the invention.
Figures 5A-C illustrate examples of test stimuli and masks presented to a user in an embodiment of the present invention in which the user's visual impairment is assessed.
Figures 6A-C illustrate further variations in the stimuli and masks of Figures 5A-C.
Figure 7A is a graph showing mean error rate plotted against exposure duration (i.e. test stimulus exposure duration) as a function of Mini Mental State Examination score according to an embodiment of the present invention using the test stimuli of Figure 1. Figure 7B is a graph showing mean error rate plotted against exposure duration (i.e. test stimulus exposure duration) as a function of Mini Mental State Examination score according to an embodiment of the present invention using the test stimuli of Figure 3.
Figure 8 is a graph showing mean response time plotted against exposure duration (i.e. test stimulus exposure duration) as a function of Mini Mental State Examination score according to an embodiment of the present invention using the test stimuli of Figure 1.
Figure 9A is a graph showing mean error rate plotted against exposure duration (i.e. test stimulus exposure duration) as a function of HIV Dementia Scale score according to an embodiment of the invention using the test stimuli of Figure 1. Figure 9B is a graph showing mean response time plotted against exposure duration (i.e. test stimulus exposure duration) as a function of HIV Dementia Scale score according to an embodiment of the present invention for the test stimuli of Figure 1.
Figures 10A and 10B are graphs showing the mean error rate and response time respectively, plotted against exposure duration (i.e. test stimulus exposure duration) as a function of partial sleep deprivation according to an embodiment of the present invention using the test stimuli of Figure 3.
Table 1 shows inter-trial correlations for both error rate and response time for use of the method on 9 consecutive working days.
Table 2A shows the absence of correlations between error rate and pre-morbid IQ and depression according to the present method.
Table 2B shows the absence of correlations between response time and pre-morbid IQ and depression according to the present method.
Table 3A shows the concordance between error rate and standard measures of cognitive performance according to an example of the method.
Table 3B shows the concordance between response time and standard measures of cognitive performance according to an example of the method .

### Detailed Description

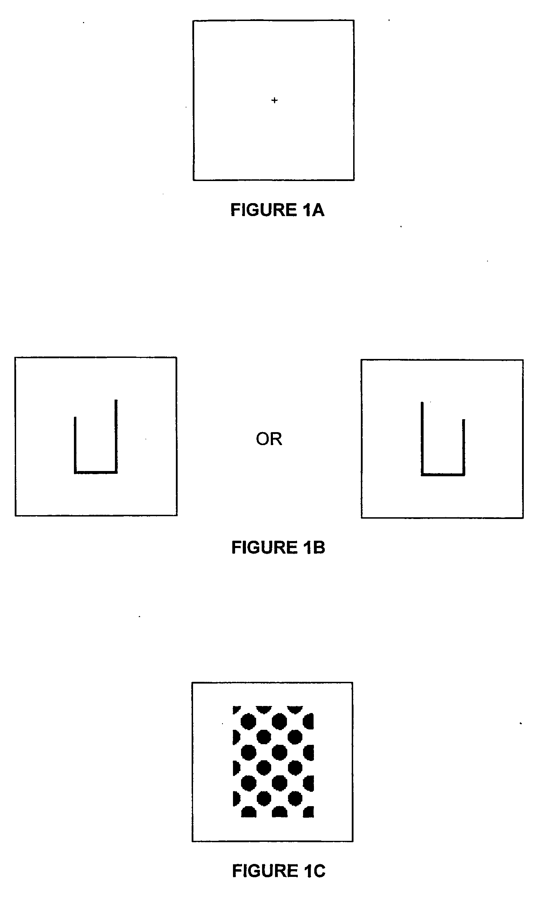

The method disclosed involves first presenting a user with a with a focal point stimulus in the form of a small centrally located cross, as illustrated in Figure 1A. A visual test stimulus is then presented for a predetermined test stimulus exposure duration. The visual test stimulus may be of any kind. In one example, the visual test stimulus is in the form of two parallel lines of different length which are joined, such as the ones illustrated in Figure 1B. The images in Figure 1B are presented one at a time for a relatively short period known as the test stimulus exposure duration and are replaced at the end of each test stimulus exposure duration by a mask consisting of filled circles. The focal point stimulus, then the test stimulus and then the subsequent mask are repeatedly presented to the user for pre-determined test stimulus exposure durations which are typically chosen to be between 10ms and 300ms. The focal point stimulus and mask can be presented for any duration which is longer than the longest test stimulus exposure duration. In a preferred example, the focal point stimulus is presented for 350ms, although anywhere between 300ms and 2 seconds may be suitable. The user then responds to the visual test stimulus at the end of each test stimulus exposure duration by pressing a button to indicate their perception of a particular characteristic of the stimulus.

For Figure 1B, the shorter line is presented on the left and right sides, preferably an equal number of times and preferably at random. The user provides a response which is indicative of whether the short line is on the left or right hand side of the visual stimulus area. A computer may be used to record each of the user's responses together with the length of time taken for the user to respond to each stimulus presented. This length of time is termed the "response time".

The data obtained for each test stimulus exposure duration are then pooled to obtain a mean error rate and a mean response time. These mean values for each test stimulus exposure duration can be used to form an error rate curve or a response time curve which may be compared to a reference curve previously generated using data from a population of cognitively normal individuals. Alternatively, the reference curve may represent data produced by the same user in a previous assessment or a series of previous assessments. This may be particularly useful for monitoring changes in cognitive impairment or cognitive ability.

The inventors have found that significantly slower response times or significantly higher error rates for one or more of the test stimulus exposure durations tends to be indicative of cognitive impairment. The error rate curve based on the error rate data can be used to improve the sensitivity of the inventive method. Shifts in the response time curve relative to a reference response time curve can provide a highly sensitive measure of cognitive impairment. The extent of any deviation away from the reference curve can be used to assess the extent of cognitive impairment and can be used to detect very subtle changes.

Figure 1 illustrates an example of the method wherein frames of a 200x200 pixel graphic are presented to the user in the centre of a 15 inch flat screen monitor at a resolution of 600x480. Figure 1A illustrates a focal point stimulus which is presented to the user before commencing the task of being presented with and responding to visual test stimuli. The user is asked to maintain their visual focus on the focal point while the test stimuli (as shown in Figure 1B) are presented to the user. Each of these test stimuli can be individually presented for various durations, such as: 13, 26, 39, 52, 65, 91, 117, 143, 169, 195 or 221 ms. After presentation of each test stimulus and at the end of the test stimulus exposure duration the stimulus is replaced with a mask such as the one shown in Figure 1C. The user nominates "left" or "right" as their response to each stimulus, depending on whether the stimulus presented has a shorter line on the left or the right of the screen. The user's response is then recorded and compared to the actual stimulus presented to determine whether their response was correct or incorrect. The time taken for the user to respond (measured from presentation of the stimulus to the user's provision of a response) is also measured. A user profile can then be developed by compiling the user's responses to the stimuli and the time taken to respond in each case.

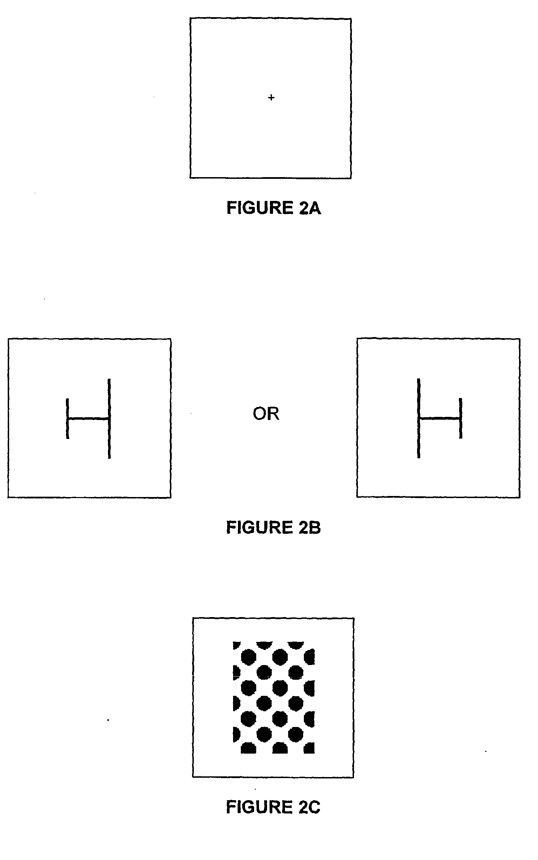

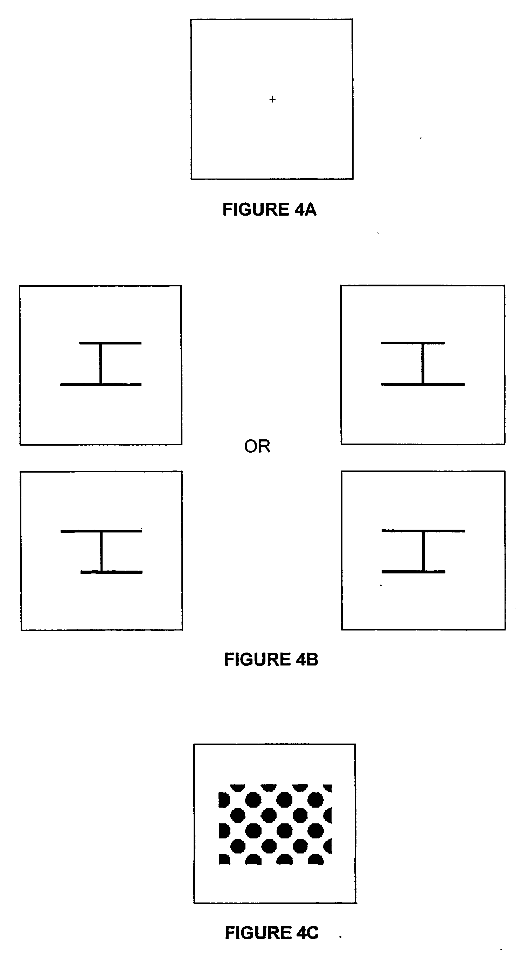

Figures 2, 3 and 4 each illustrate examples of the method with alternative test stimuli. Generally, a user will be tested using the same set of visual test stimuli repeatedly. However, different stimuli may be used to test users in groups with different levels of cognitive ability. For example, an elderly user may be assessed using the visual test stimuli of Figure 1 B whereas a pilot or other cognitively advanced subject may be assessed using more complex visual test stimuli such as the stimuli of Figure 4B. The stimuli need not be limited to visual representations of lines. Other forms of visual stimulus may be used which include one or more of colour, texture, curves and other non-linear forms.

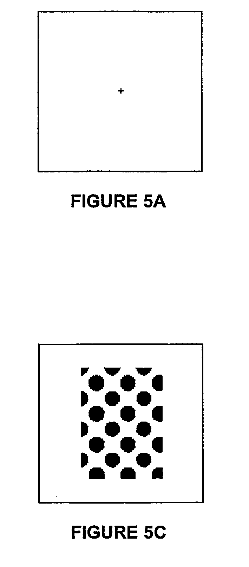

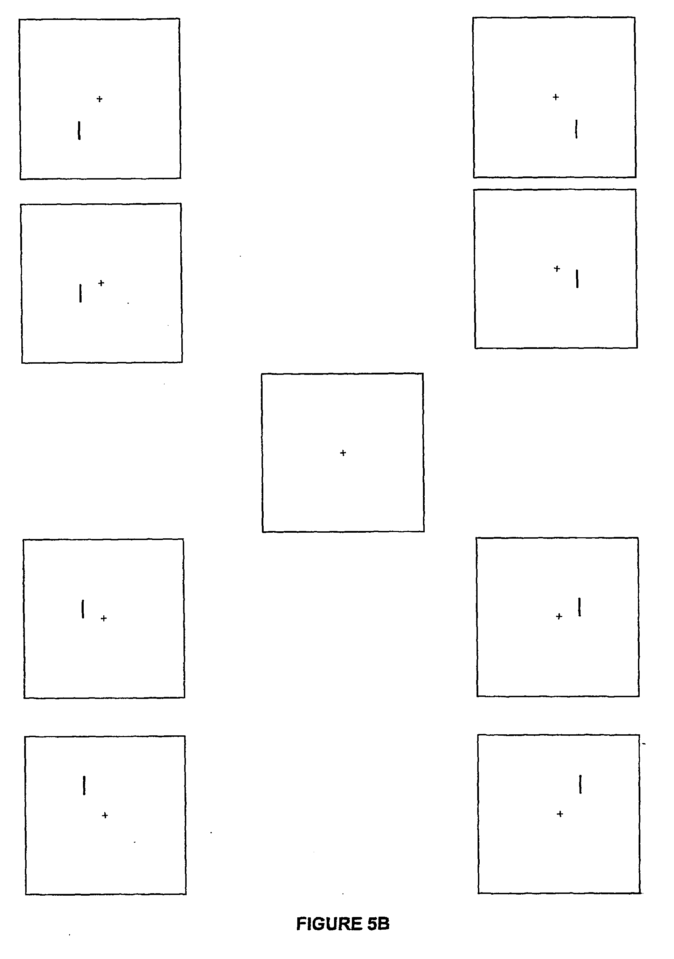

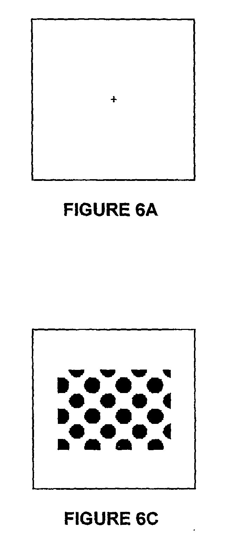

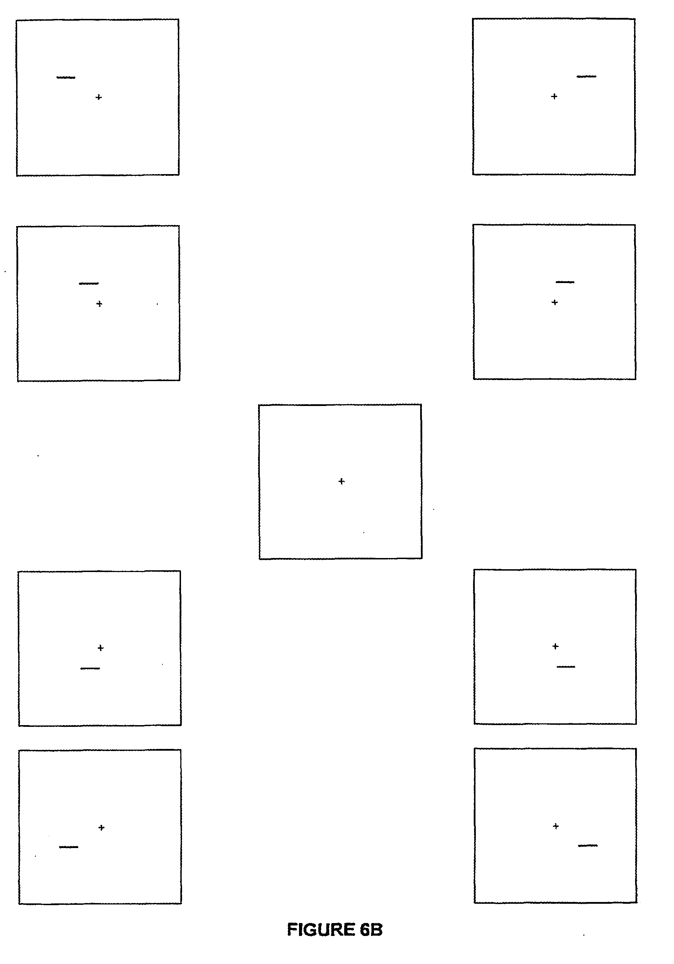

Figure 5 illustrates a sequence of visual stimuli presented to the user to assess visual impairment. Firstly, the focal point stimulus shown in Figure 5A is presented in the form of a small, centrally located cross. Then, one of the test stimuli shown in Figure 5B is presented for a predetermined duration of between 10-300 ms followed by the presentation of the mask shown in Figure 5C. The user is presented with instructions and informed that their task is to decide whether a small black line appears on the screen. It may appear on the left or the right of the focal point stimulus, and/or above or below the focal point stimulus. The user then responds by pressing a button to indicate whether they have detected the presence of the test stimulus (i.e. the black line). A correct response is provided when the user presses the button when a line is presented, or provides no response when a line is not presented. In one example 50% of the trials included the focal point stimulus and a line and in the other 50% the focal point only is presented (i.e. the test stimulus does not include a line). The user's response is then used to create a user profile which can be matched against a reference profile to determine the degree of visual impairment. Figure 6 depicts an alternative set of visual test stimuli which uses horizontal test stimuli.

In some cases, a user may be able to detect the presence of the visual stimulus in some regions relative to the focal point stimulus, but not in others. Therefore, this example may be used to detect subtle visual field deficits at a retinal or a cortical level. This reduces the likelihood of a user being incorrectly classified as having a cognitive impairment when an incorrect response to presentation of a test stimulus is the result of visual impairment, addressing a major deficiency of prior art methods.

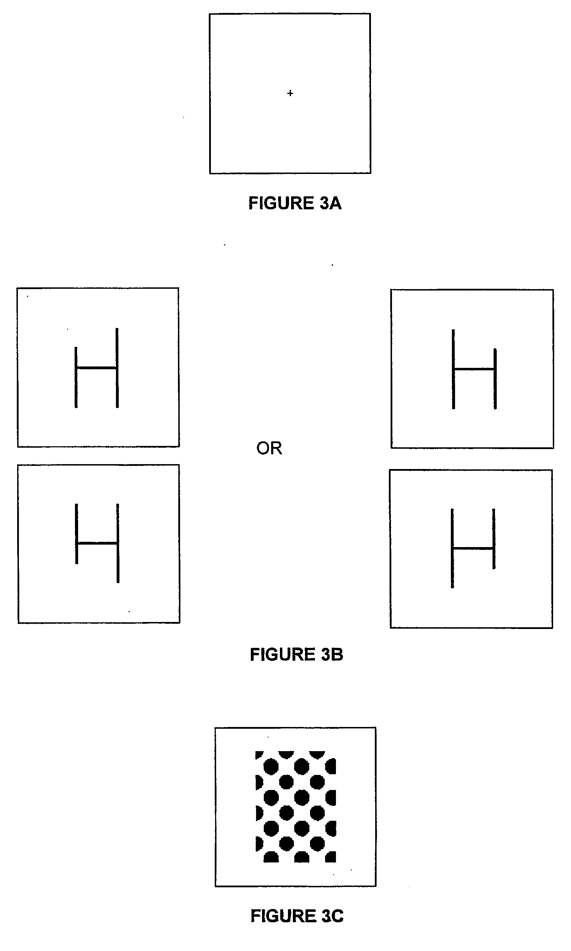

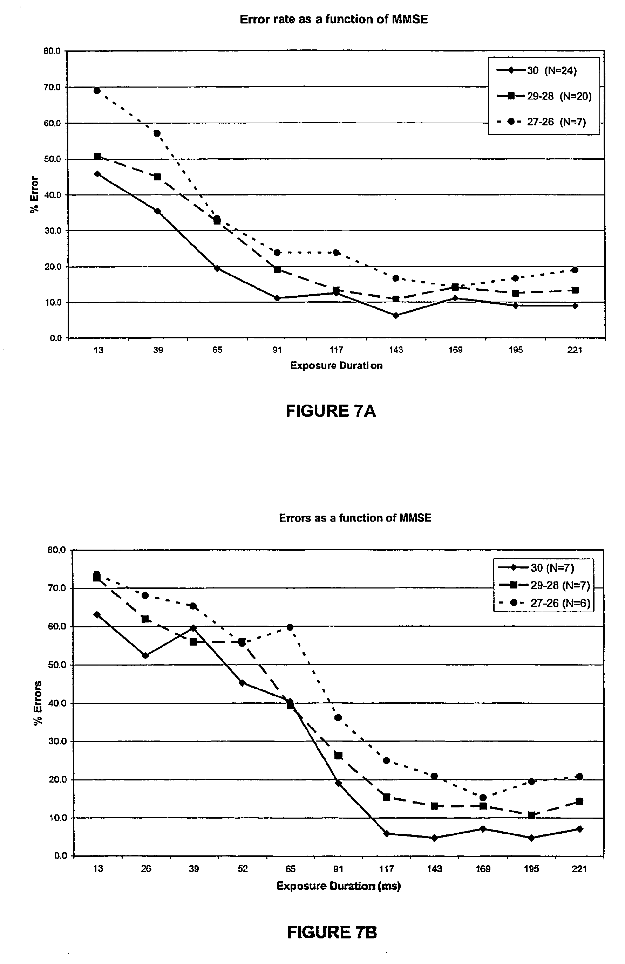

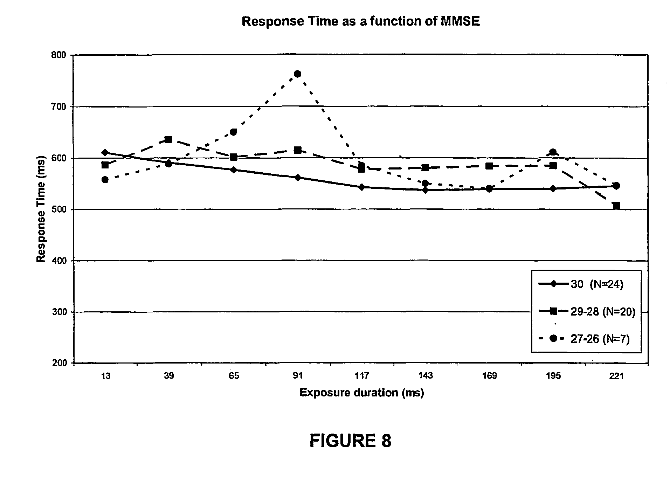

Figure 7A and B are graphs that plot the mean error rate (%) against test stimulus exposure duration as a function of score on the Mini Mental State Exam (MMSE) for elderly participants (65-85 years), using stimuli such as those illustrated in Figure 1 (7A) and those illustrated in Figure 3 (7B). A Mini Mental State Exam (MMSE) score of 30 is judged to be normal while lower MMSE scores of 29-26 show subtle cognitive impairment. Figure 8 shows another graph which charts the mean response time against the test stimulus exposure duration. Again, a MMSE of 30 is shown as a normal indicator while deviations from the normal of MMSE's corresponding to 29, 28, 27 and 26 show subtle cognitive impairment.

The method is advantageous in that it provides a method of detecting subtle cognitive impairment that can be applied to individuals who are considered high functioning individuals with an almost normal score of between 26-29 on a MMSE. This results from the ability to detect differences in individuals who differ by only small increments on the MMSE.

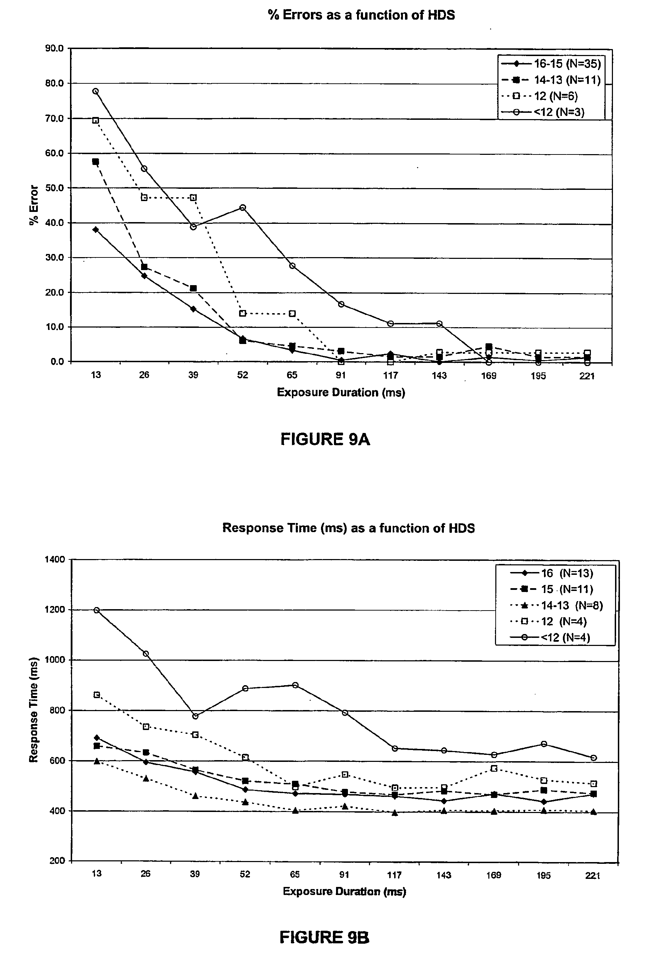

The method is also advantageous in that it provides a method of assessing cognitive impairment amongst individuals who are seropositive for HIV-1. Figures 9A and B are graphs that plot the mean error rate (%) and Mean response times against test stimulus exposure duration as a function of scores on HIV Dementia Scale (HDSE), using stimuli such as those illustrated in Figure 1. The HDS is a diagnostic instrument in which a score of 16 reflects cognitively normal performance while a score of 10 or less indicates the presence of HIV Dementia.

The method is also advantageous in that it may be performed relatively quickly over a period of around 5-10 minutes and does not require a separate training session. The method also requires no supervision from highly trained personnel. Therefore, there is no need for the method to be performed by a trained psychologist. In fact, when the method is performed using a computer, it may be automated and the user assessed without supervision.

The inventors have also found that the method measures the subconscious cognitive processes in a way that is substantially free from the influence of a learning effect. Because of this, and the fact that the method can be performed quickly, the subjects can be re-evaluated by the method several times in a day if necessary.

The inventors have found that data from individual users of the test show a high degree of test-retest reliability. Table 1 shows inter-trial correlations for five users who were tested daily for 9 days.

A further advantage is that the method generates quantitative data that does not involve subjective interpretation. The results of the method can therefore be compared to results gathered by others where comparison of results is necessary. Further, a series of assessments made over a period of time can be used to form a history, showing variation in cognitive ability in a user.

The inventors have also found that the method is not biased by pre-morbid intelligence or depression. Tables 2A,B summarise data obtained from 42 users who were seropositive for HIV-1. No correlations were found between measures on the present method and measures obtained on the Beck Depression Inventory and the National Adult Reading Test (NART).

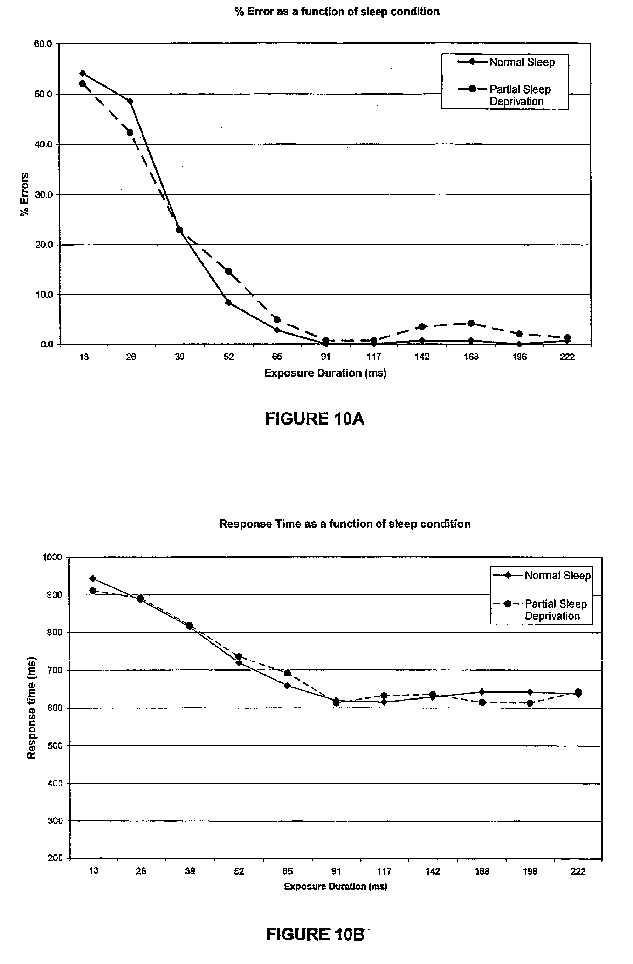

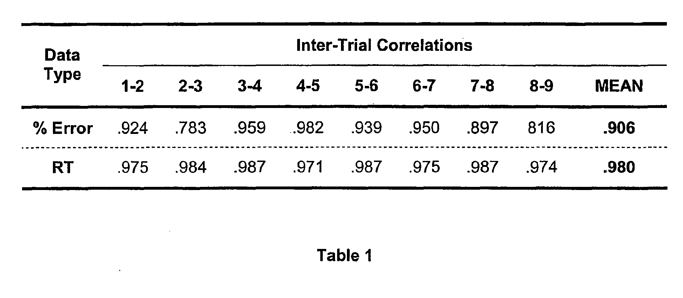

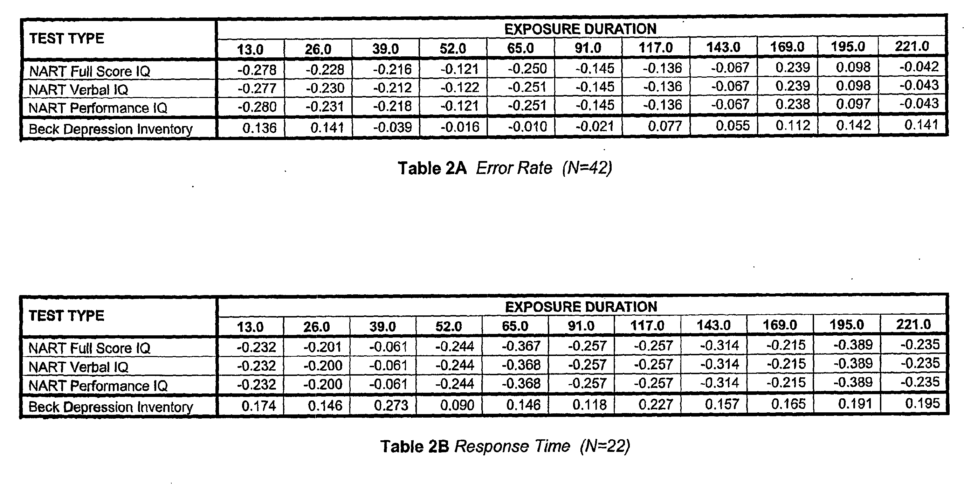

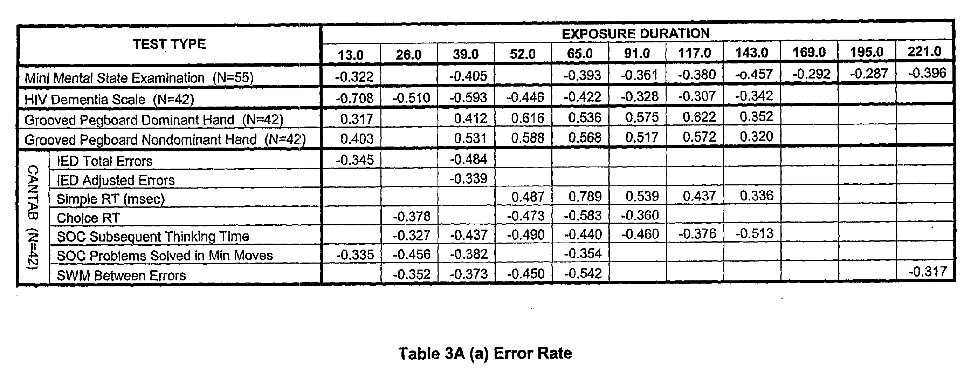

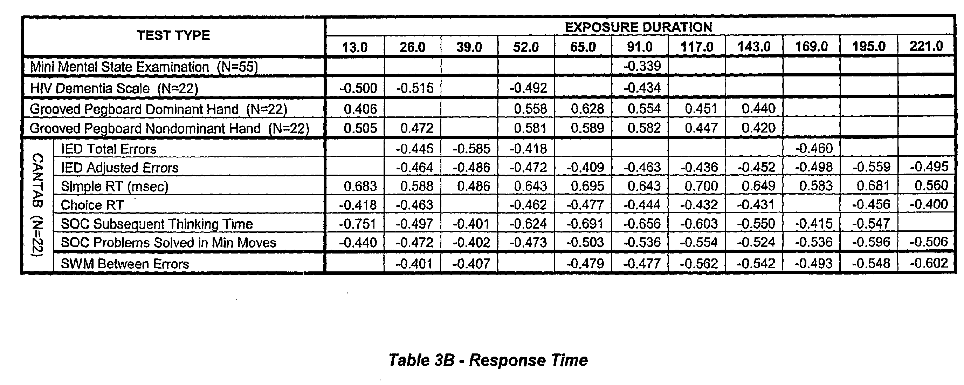

The inventors have also found that the method is not biased by mild sleep deprivation. Figures 10A,B summarise data obtained from 12 users who had been subjected to a 40% reduction in normal sleep hours for two consecutive nights. No significant difference were found between their performance when sleep deprived or normally rested, for either response time or error rate (p<.05), despite a significantly worse performance on the Psychomotor Vigilance Task (p<.05), a test known for its sensitivity to sleep loss.

The method is advantageous in that unlike many other measures of cognitive impairment, the method does not have a ceiling effect. In more than 600 trials of the test no one has yet achieved a perfect score for response time or error rate.

The inventors have also found that the method has a high degree of concurrent validity when compared against gold standard measures of cognitive impairment. Tables 3A,B summarise data obtained from a group of 55 elderly (65-85 y.o.) users and a group of 42 users who were seropositive for HIV-1. High correlations (p<.05) were found for response time and error rate when compared to measures on the Mini Mental State Examination (elderly users) and HIV Dementia Scale, the Grooved Pegboard and components of the CANTAB (HIV-1 user).

The method can be implemented in any suitable manner. In one envisaged form the method would be implemented through the use of a computer program operating on a standard platform such as Microsoft Windows™.

This invention has potential application in a wide variety of circumstances and is particularly applicable to detection of subtle cognitive impairment or in the re-evaluation of test subjects for changes in the degree of cognitive impairment. A person skilled in the art will realise that some possible, but not necessarily exclusive applications, of the present invention include:
(a) Measurement of cognitive performance before and after major surgical procedures, such as cardiac surgery, to detect subtle neurological complications that often arise from such procedures;
(b) Quantification of the degree and rate of recovery from instances of trauma such as head trauma from sporting or vehicle related injuries;
(c) Monitoring of the cognitive side-effects of drugs used to treat a variety of illnesses;
(d) Quantitative measurement and comparison of the effectiveness of different pharmaceutical regimes in the treatment of neurological conditions;
(e) Precautionary monitoring of mild cognitive impairment in ostensibly high functioning individuals in whom mild cognitive impairment may have serious consequences. This occurs in professions such as airline or fighter pilots and air traffic controllers.
(f) Detection and monitoring of cognitive decline that may be associated with the onset of a progressive neurological condition such as Alzheimer's disease.
(g) Detection and monitoring of transient cognitive impairment resulting from intoxication, including intoxication caused by drugs and/or alcohol.
(h) Detection and monitoring of transient cognitive impairment resulting from acute sleep deprivation.

It is to be understood that various additions, alterations and/or modifications may be made to the parts previously described without departing from the ambit of the Invention which is defined by the appended claims.

## Claims

1. A system for assessing cognitive impairment of a user, including:
(a) presentation means for presenting a visual test stimulus (Fig. 1b) to a user for a range of pre-determined test stimulus exposure durations;
(b) mask means (Fig. 1c) for masking the visual test stimulus after presentation of the test stimulus to the user, wherein the mask includes an image having a plurality of filled circles or curved lines or parts thereof;
(c) response measuring means to measure the response from the user after presentation of the visual test stimulus;
(d) processing means to process the response from the user over a range of pre-determined test stimulus exposure durations to develop the user profile over the range; and
(e) assessment means to compare the user profile to a reference profile to assess cognitive impairment in the user.

2. A system for assessing cognitive impairment of a user according to claim 1 further including focal point presentation means for presenting a focal point stimulus to the user.

## Patentansprüche

1. System zum Beurteilen einer kognitiven Beeinträchtigung eines Benutzers, das Folgendes beinhaltet:
(a) Präsentationsmittel zum Präsentieren eines visuellen Prüfreizes (Fig. 1b) für einen Benutzer für einen Bereich von vorbestimmten Prüfreizexpositionsdauern;
(b) Maskenmittel (Fig. 1c) zum Maskieren des visuellen Prüfreizes, nachdem der Prüfreiz dem Benutzer präsentiert worden ist, wobei die Maske ein Bild mit mehreren gefüllten Kreisen oder gekrümmten Linien oder Teilen davon enthalt;
(c) Reaktionsmessmittel zum Messen der Reaktion von dem Benutzer nach der Präsentation des visuellen Prüfreizes;
(d) Verarbeitungsmittel zum Verarbeiten der Reaktion von dem Benutzer über einen Bereich von vorbestimmten Prüfreizexpositionsdauern, um das Benutzerprofil über den Bereich hinweg zu entwickeln; und
(e) Beurteilungsmittel zum Vergleichen des Benutzerprofils mit einem Referenzprofil, um eine kognitive Beeinträchtigung in dem Benutzer zu beurteilen.

2. System zum Beurteilen einer kognitiven Beeinträchtigung eines Benutzers nach Anspruch 1, weiterhin mit Brennpunktpräsentationsmitteln, um dem Benutzer einen Brennpunktreiz zu präsentieren.

## Revendications

1. Système d'évaluation de déficience cognitive d'un utilisateur comprenant :
(a) un moyen de présentation d'un stimulus de test visuel (Fig. 1b) à un utilisateur pendant une plage de durées prédéterminées d'exposition à un stimulus de test,
(b) un moyen de masque (Fig. 1c) pour masquer le stimulus de test visuel après présentation du stimulus de test à l'utilisateur, le masque comprenant une image ayant une pluralité de cercles pleins ou de lignes courbes, ou de parties de ceux-ci ;
(c) un moyen de mesure de réponse pour mesurer la réponse de l'utilisateur après présentation du stimulus de test visuel ;
(d) un moyen de traitement pour traiter la réponse de l'utilisateur sur une plage de durées prédéterminées d'exposition à un stimulus de test pour développer le profil de l'utilisateur sur la plage ; et
(e) un moyen d'évaluation pour comparer le profil de l'utilisateur à un profil de référence pour évaluer la déficience cognitive de l'utilisateur.

2. Système d'évaluation de déficience intellectuelle d'un utilisateur selon la revendication 1, comprenant en outre un moyen de présentation de point focal pour présenter un stimulus de point focal à l'utilisateur.
